Europäisches Patentamt

**European Patent Office**

Office européen des brevets

(11) Publication number: **0 168 217**
A2

(12) **EUROPEAN PATENT APPLICATION**

(21) Application number: **85304781.9**

(22) Date of filing: **04.07.85**

(51) Int. Cl.⁴: **C 07 K 3/02**
**C 12 P 21/00**
**//(C12P21/00, C12R1:91)**

(30) Priority: **12.07.84 JP 145139/84**

(43) Date of publication of application:
**15.01.86 Bulletin 86/3**

(84) Designated Contracting States:
**CH DE FR GB IT LI SE**

(71) Applicant: **MAKITSUBO, Takeshi**
**Room 803 Touei Bldg 176 Fujigaoka**
**Meito-ku Nagoya-shi Aichi 465(JP)**

(72) Inventor: **MAKITSUBO, Takeshi**
**Room 803 Touei Bldg 176 Fujigaoka**
**Meito-ku Nagoya-shi Aichi 465(JP)**

(74) Representative: **Myerscough, Philip Boyd et al,**
**J.A.Kemp & Co. 14, South Square Gray's Inn**
**London, WC1R 5EU(GB)**

(54) Method of extracting tumor necrosis factor-like substance.

(57) Disclosed herein is a method of extracting a large amount of pure tumor necrosis factor-like substance from fibroblast, epithelial or lymphoblast-like cells killed by an overpopulation in a culture medium or destroyed in a culture medium by an artificial treatment such as a quick freezing and quick thawing. The pure tumor necrosis factorlike substance has the cytotoxicity or cytostaticity to tumor cells.

EP 0 168 217 A2

Croydon Printing Company Ltd

0168217

## METHOD OF EXTRACTING TUMOR NECROSIS FACTOR-LIKE SUBSTANCE

This invention relates to a simple method of abundantly extracting tumor necrosis factor-like substance (TNS), which exerts an effective cytotoxicity against tumor cells instead of normal cells. This TNS has a quite similar characteristics of previously known tumor necrosis factor (TNF) in vivo or vitro.

There are many reports of substances which are produced by a few gram-positive bacterias or endotoxins, and have a physiological activity as well as a cytotoxic activity (CTA) to tumor cells. Dr. Carrle et al (J. Exp. Med. 142:1600-1605, 1975), for example, found that a cytostatic factor for tumor cells was obtained from the peritoneal exudate cells of a normal rat treated with endotoxin. Thereafter, they reported in Nature (London), 273:758-759, 1978 that an aforementioned cytostatic factor was arginase. Dr. Reed et al reported in J. Immunol., 115:395-404, 1975 that a protein consisted of a molecular weight of 45.000 daltons was more cytotoxic against cultured tumor cells. This protein was in supernatant produced from cultured cells of an endotoxin treated rat or human. Dr. Carswell et al [Proc. Natl. Acad. Sci. (USA), 72(9):3666-3670, 1975] reported a phenomena that serum from BCG (Bacillus Calmette-Guerin) infected Swiss mouse (CD-1) treated with intravenously injected endotoxin is cytotoxic on cultured L-cells of Fl mouse (Balb/c x C57BL/6) transplanted Meth A sarcoma, and this serum was named as TNF.

TNF of Dr. Carswell, however, necessitated a number of animals in order to extract TNF, and it from each animal was necessarily uniform. It consisted of several kinds of serum protein. Processes to extract a small amount of TNF from serum were complex and consuming. The method of extracting TNF, so far, have not always available in procedures or quantity.

0168217

The objects of this invention are to extract TNS simply and a lot in quantity. The method is the following:

(1)  Maintain in culture a sort of cell of a fibroblast, epithelial or lymphoblast-like cell.

(2)  Kill the cultured cell, naturally or artificially.

(3)  Extract TNS from the killed cell.

The inventor of this invention produced in success a large amount of pure TNS from cultured L-929 fibroblasts, and found that TNS was present in supernatant obtained from centrifugation of fibroblast-like cells after step (1) and (2). The following is described in details; as step (a), a macrophage cell line of CAMU-3-R or a fibroblast cell line of L-929 are homogeneously killed in culture medium. As step (b), the medium in step (a) is mixed with a tumor cell line of Meth A, and this cells are incubated for 48 hours. As step (c), the culture medium containing the killed fibroblast cell line is mixed and incubated with the tumor cell lines of a Meth A for 48 hours. As step (d), the killing effect of the fluid in step (c) which contains a macrophage cell line is compared with that of fibroblast cell lines. This percent index means the cytotoxicity or cytostaticity to tumor cells. The percent indices of the macrophage, fibroblast and artificially destroyed cell lines are 8.8%, 83.7% and 96.9%, respectively. The fluid of a higher percentage was not cytotoxic to normal cells. This result was confirmed by a production of a large amount of TNS in cells in a fibroblast, an epithelial and a lymphoblast-like cell. The TNS extraction from fibroblast, epithelial and lymphoblast-like cells is significant.

Culture of fibroblast, epithelial or lymphoblast-like cells obtained in vivo is more simple than of cultured macrophages. These cells are more productive without consuming time than macrophages. TNS from a definite cell is more pure than that from animals. Next, this invention confirmes a biological role of the TNS as a curable drug for cancer.

The following procedures are employed as this invention.

[I]  Make-up of TNS

(1)  Culture epithelial cells of a CAMU-3-A7 cell in a petri dish.

(2)  Detach CAMU-3-A7 cells on petri dishes using tripsin or EDTA.

(3)  Mix the cultured CAMU-3-A7 cells with culture medium such as MEM, RPMI1640 or KN7 including 5% newborn calf serum, and adjust the cells to the number of $3 \times 10^6$/ml.

(4)  Culture the cell suspension in [1]-(3) for 96 hours in 5% $CO_2$-air at 37°C, and allow to kill the cells by overpopulation.

(5)  Centrifuge this killed CAMU-3-A7 cells at 2000r.p.m. for 10 minutes, and collect the supernatant, which may contains TNS and filtered through a 0.45$\mu$m filter.

[II]  Identification of TNS

(1)  Adjust the number of the tumor cells of a Meth A sarcoma of a mouse to $4 \times 10^5$/ml tumor cells the added 5% newborn calf serum culture medium.

(2)  Mix the solution of [II]-(1) with the same amount of the [I]-(5) solution.

(3)  Incubate the solution [II]-(2) for 48 hours in 5% $CO_2$-air at 37°C.

(4)  Count the number of live tumor cells in [II]-(3) stained with trypan blue.

The positive relationship between the concentration of supernatant (TNS) and CTA is noted. CTA, that is, is parallel to dosage of TNS.  See Table 1.

Table 1

| Culture Medium with Supernatant in Concentration Ratio of | 48 Hour-CTA of a Meth A Sarcoma | |
|---|---|---|
| | Number of Living Cells $(\times 10^4)$ | CAT (%) |
| 1 to 0 | 68 | 0 |
| 0.9 to 0.1 | 32.7 | 44.6 |
| 0.8 to 0.2 | 14.0 | 79.4 |
| 0.7 to 0.3 | 8.2 | 87.9 |
| 0.6 to 0.4 | 6.7 | 90.1 |
| 0.5 to 0.5 | 5.6 | 91.8 |

[III]  Biological characteristics of TNS

(A)  The supernatant [I]-(5) was heated at 56°C for 30 minutes, at 70°C for 60 minutes, and at 85°C for 15 minutes, respectively.  See Table 2.  The negative relationship between CTA and heating was noted.

Table 2

| Heating Conditions of Supernatant | 48 Hour-CTA of a Meth A Sarcoma | |
|---|---|---|
| | Number of Living Cells $(\times 10^4)$ | CTA (%) |
| at 56°C for 30 Minutes | 14.2 | 76.3 |
| at 70°C for 60 Minutes | 49.5 | 17.5 |
| at 85°C for 15 Minutes | 43.0 | 28.3 |
| Culture Medium without Supernatant, without a Heating Treatment | 60 | 0 |

This result indicates that TNS extracted from CAMU-3-A7 is likely to be a glycoprotein, because CTA decline with heating, especially more than 50°C.

(B)   CTA according to concentrations of CAMU-3-A7 in cell suspension.   (1) Prepare, four sets of CAMU-3-A7 cell suspension contaning $1 \times 10^6$/ml, $2 \times 10^6$/ml, $3 \times 10^6$/ml and $4 \times 10^6$/ml cells and culture them for 96 hours, (2) Kill the suspended cells by over population, (3) Centrifuge and isolate the supernatant, (4) Culture each batches of supernatants for 48 hours, the sarcoma Meth A cell in the medium contaning, and (5) calculate CTA.

The result of this experiment indicates that the increased number of suspended cells is parallel to CTA. See Tables 3A and 3B.

Table 3A

|  | CTA According to Concentrations of CAMU-3-A7 in Cell Suspension | |
| --- | --- | --- |
|  | Number of Living Cells ($\times 10^4$) | CTA (%) |
| Culture Medium without Supernatant | 63.5 | 0 |
| Concentration of CAMU-3-A7 Cell Suspension $1 \times 10^6$/ml | 23.1 | 63.6 |
| Concentration of CAMU-3-A7 Cell Suspension $2 \times 10^6$/ml | 18.9 | 70.2 |

Table 3B

|  | CTA According to Concentrations of CAMU-3-A7 in Cell Suspension | |
| --- | --- | --- |
|  | Number of Living Cells ($\times 10^4$) | CTA (%) |
| Culture Medium without Supernatant | 80 | 0 |
| Concentration of CAMU-3-A7 Cell Suspension $3 \times 10^6$/ml | 3.1 | 96.1 |

- 6 -

0168217

Table 3B
(continued)

| | CTA According to Concentrations of CAMU-3-A7 in Cell Suspension | |
|---|---|---|
| | Number of Living Cells (x$10^4$) | CTA (%) |
| Concentration of CAMU-3-A7 Cell Suspension 4x$10^6$/ml | 1.3 | 98.4 |

The explanation about [II] is as follows: [II-1] Epithelial like-cells of a CAMU-3-A7 cells cultured in a petri dish. [II-2] Detach cells from the petri dish face using tripsin or EDTA. [II-3] mix the culture cells in culture medium such as MEM, RPMI1640 or KN7 contaning 5% newborne calf serum and adjust the cell concentration of 3x$10^6$/ml in suspension. [II-4] Put the cell suspension into a cryotube and freeze quickly in liquid nitrogen and keep it for 5 minutes. [II-5] Thaw it in 37°C hot water for 10 minutes. [II-6] Repeat [II-4] and [II-5] three times. [II-7] Centrifuge the solution [II-6] at 2000 r.p.m. for 10 minutes, and separate the supernatant, which may contain TNS.

In comparison of TNS [I] and TNS [II], each CTA was identical and the same CTA was obtained from the supernatant of the lymphoblast-like cell (RL$-1) or the fibroblast-like cell (L929) instead of CAMU-3-A7. See Table 4.

Table 4

| | Comparison between CTA of TNS [I] and TNS[II] | |
|---|---|---|
| | Number of Living Cells (x$10^4$) | CTA (%) |
| Culture Medium without Supernatant | 78 | 0 |
| Culture Medium with Supernatant Contained Killed CAMU-3-A7 Cells | 2.4 | 96.9 |

Table 4
(Continued)

|  | Comparison between CTA of TNS [I] and TNS [II] | |
|  | Number of Living Cells (x10^4) | CTA (%) |
| --- | --- | --- |
| Culture Medium with Supernatant Contained Destroyed CAMU-3-A7 Cells | 1.3 | 98.3 |
| Culture Medium with Supernatant Contained Killed L929 Cells | 12.7 | 83.7 |

The followings represent about characteristics of fibroblast, epithelial or lymphoblast-like cells:

(A) Fibroblast-like cells which adhere on a glass face of a petri dish form a fusiform style, when they are separated from in vivo and are isolated by using tripsin or EDTA (Ethylene Diamine Tetra Acetate).

(B) Epithelial-like cells which adhere on a glass face of a petri dish form a polyhedral style, when they are separated from in vivo and are isolated by using tripsin or EDTA.

(C) Lymphoblast-like cells which partly adhere on a glass face of a petri dish drift in culture medium and form a sphere style, when they are separated from in vivo and are isolated by using tripsin or EDTA.

Most of fibroblast-like cells, epithelial-like cells or lymphoblast-like cells have few characteristics such as Fc receptor, $C_3$ receptor or phagocytosis, a cytotoxic activity on tumor cells, which macrophages normally have. Cells having a tumorigenicity which are produced by tumor viruses or cancerogenic substances are classified into the same kind of fibroblast-like cells, epithelial-like cells or lymphoblast-like cells.

The following experiment was tried to confirm a molecular weight of TNS produced from a CAMU-3-A7 adjusted to $3 \times 10^6$/ml

and destroyed by a quick freezing and thawing. A special membrane which can be filtered less than a molecular weight of $1 \times 10^4$, $3 \times 10^4$ or $1 \times 10^5$ respectively was used in order to confirm the molecular weight of TNS in the supernatant. Table 5 shows the results of this experiment.

Table 5

|  | Comparison of CTA on Tumor Cells of a Meth A Sarcoma Cultured for 48 Hours. | |
| --- | --- | --- |
|  | Number of Living Cells ($\times 10^4$) | CTA (%) |
| Culture Medium without Supernatant | 77 | 0 |
| Less Than Molecular Weight of $1 \times 10^4$ | 66 | 14.3 |
| Less Than Molecular Weight of $3 \times 10^4$ | 67 | 13.0 |
| Less Than Molecular Weight of $1 \times 10^5$ | 77.5 | 0 |
| More Than Molecular Weight of $1 \times 10^5$ | 4.0 | 94.8 |

As is evident from Table 5, the molecular weight of TNS is expected to be more than $1 \times 10^5$.

The following experiment was tried to confirm CTA on tumor cells of a Meth A sarcoma cultured for 48 hours in a culture medium mixed with a supernatant produced from several kinds of cells. The results of this experiment are shown in Tables 6 to 8.

Table 6

| | Comparison of CTA on Tumor Cells of a Meth A Sarcoma Cultured for 48 Hours, which Are Produced from Several Kinds of Cells | |
| --- | --- | --- |
| | Number of Living Cells (x10$^4$) | CTA (%) |
| Culture Medium without Supernatant | 84.5 | 0 |
| Culture Medium with Supernatant Produced from a CAMU-3-A7 Adjusted to 3x10$^6$/ml Cultured for 96 Hours | 1.1 | 98.7 |
| Culture Medium with Supernatant Produced from a Meth A Sarcoma Adjusted to 3x10$^6$/ml and Destroyed by a Quick Freezing and Thawing | 77.5 | 8.3 |

Table 7

| | Number of Living Cells (x10$^4$) | CTA (%) |
| --- | --- | --- |
| Culture Medium without Supernatant | 74 | 0 |
| Culture Medium with Supernatant Produced from a Meth A Sarcoma Adjusted to 3x10$^6$/ml and Destroyed by a Quick Freezing and Thawing | 84 | 0 |
| Culture Medium with Supernatant Produced from a RL♂1 Adjusted to 3x10$^6$/ml and Destroyed by a Quick Freezing and Thawing | 6.0 | 91.9 |

Table 7
(Continued)

|  | Number of Living Cells (x10^4) | CTA (%) |
|---|---|---|
| Culture Medium with Supernatant Produced from a SV-T2 Adjusted to 3x10^6/ml and Destroyed by a Quick Freezing and Thawing | 64.0 | 13.5 |

Table 8

|  | Number of Living Cells (x10^4) | CTA (%) |
|---|---|---|
| Culture Medium without Supernatant | 83.5 | 0 |
| Culture Medium witn Supernatant Produced from a L929 Adjusted to 3x10^6/ml and Destroyed by a Quick Freezing and Thawing | 2.0 | 97.6 |
| Culture Medium with Supernatant Produced from a Balb/3T3 Adjusted to 3x10^6/ml and Destroyed by a Quick Freezing and Thawing | 1.8 | 97.8 |
| Culture Medium with Supernatant Produced from a Normal Mouse Fetus Adjusted to 3x10^6/ml and Destroyed by a Quick Freezing and Thawing | 20.7 | 75.2 |
| Culture Medium with Supernatant Produced from a YAC-1 Adjusted to 3x10^6/ml and Destroyed by a Quick Freezing and Thawing | 82.5 | 1.2 |

Table 8
(Continued)

| | Number of Living Cells (x10$^4$) | CTA (%) |
|---|---|---|
| Culture Medium with Supernatant Produced from a XC Adjusted to 3x10$^6$/ml and Destroyed by a Quick Freezing and Thawing | 81.0 | 3.0 |

As is evident from Tables 6 to 8, each supernatant contained a killed CAMU-3-A7 or a destroyed L929, RL$1, Balb/3T3 or a normal mouse fetus respectively shows a large percentage of CTA. On the other hand, each supernatant contained a destroyed Meth A sarcoma, SV-T2, YAC-1 or XC shows a small percentage of CTA.

The following experiment was tried to confirm CTA on the tumor cells of a Meth A sarcoma cultured for 48 hours in a culture medium mixed with a supernatant produced from a macrophage cell line such as CAMU-3-R or CAMU-31-R adjusted to 4x10$^6$/ml, 5x10$^6$/ml, 6x10$^6$/ml, 7x10$^6$/ml or 8x10$^6$/ml, respectively. The results of this experiment are shown in Tables 9 and 10.

Table 9

| | Comparison of CTA on the Tumor Cells of a Meth A Sarcoma Cultured for 48 Hours, in case of Using a CAMU-3-R | |
|---|---|---|
| | Number of Living Cells (X10$^4$) | CTA (%) |
| Culture Medium without Supernatant | 56.5 | 0 |
| Culture Medium with Supernatant Produced from a CAMU-3-R Adjusted to 4x10$^6$/ml | 51.5 | 8.8 |

Table 9
(Continued)

| | Comparison of CTA on the Tumor Cells of a Meth A Sarcoma Cultured for 48 Hours, in case of Using a CAMU-3-R | |
|---|---|---|
| | Number of Living Cells $(\times 10^4)$ | CTA (%) |
| Culture Medium with Supernatant Produced from a CAMU-3-R Adjusted to $5 \times 10^6$/ml | 61.8 | 0 |
| Culture Medium with Supernatant Produced from a CAMU-3-R Adjusted to $6 \times 10^6$/ml | 70.5 | 0 |
| Culture Medium with Supernatant Produced from a CAMU-3-R Adjusted to $7 \times 10^6$/ml | 59.5 | 0 |
| Culture Medium with Supernatant Produced from a CAMU-3-R Adjusted to $8 \times 10^6$/ml | 60.5 | 0 |

Table 10

| | Comparison of CTA on the Tumor Cells of a Meth A Sarcoma Cultured for 48 Hours, in case of Using a CAMU-31-R | |
|---|---|---|
| | Number of Living Cells $(\times 10^4)$ | CTA (%) |
| Culture Medium without Supernatant | 56.5 | 0 |
| Culture Medium with Supernatant Produced from a CAMU-31-R Adjusted to $4 \times 10^6$/ml | 46.5 | 17.7 |
| Culture Medium with Supernatant Produced from a CAMU-31-R Adjusted to $5 \times 10^6$/ml | 49.8 | 11.9 |

Table 10
(Continued)

|  | Comparison of CTA on the Tumor Cells of a Meth A Sarcoma Cultured for 48 Hours, in case of Using a CAMU-31-R | |
| --- | --- | --- |
|  | Number of Living Cells $(\times 10^4)$ | CTA (%) |
| Culture Medium with Supernatant Produced from a CAMU-31-R Adjusted to $6 \times 10^6$/ml | 52.8 | 6.5 |

As is evident from Table 9 or Table 10, a macrophage cell line such as CAMU-3-R or CAMU-31-R does not have an effective CTA.

The following experiment was tried to confirm CTA on normal cells separated from a minched spleen or a minched fetus of a Balb/c mouse in a gestation period cultured for 48 hours in a culture medium mixed with a supernatant produced from a CAMU-3-A7 or a L929 adjusted to $3 \times 10^6$/ml and cultured for 96 hour. The results of this experiment are shown in Table 11.

Table 11

| | Comparison of CTA on Normal Cells | | | | | |
| | Normal Cells Separated from a Spleen of Balb/c Mouse : Lymphatic-Like Cell ($1\times10^6$/ml) | | Normal Cells Separated from a Fetus of Balb/c Mouse : Lymphatic-Like Cell ($5\times10^5$/ml) | | Normal Cells Separated from a Fetus of Balb/c Mouse : Fibroblast-Like Cell ($5\times10^5$/ml) | |
| | Number of Living Cells ($\times10^4$) | CTA (%) | Number of Living Cells ($\times10^4$) | CTA (%) | Number of Living Cells ($\times10^4$) | CTA (%) |
|---|---|---|---|---|---|---|
| Culture Medium without Supernatant | 24.2 | 0 | 77.5 | 0 | 35.5 | 0 |
| Culture Medium with Supernatant Produced from a CAMU-3-A7 Adjusted to $3\times10^6$/ml and Cultured for 96 Hours | 52 | 0 | 82 | 0 | 41.3 | 0 |
| Culture Medium with Supernatant Produced from a L929 Adjusted to $3\times10^6$/ml and Cultured for 96 Hours | 55 | 0 | - | - | - | - |

As is evident from Table 11, supernatants shown in Table 11 do not have CTA on normal cells.

The following results are shown in the above mentioned experiments.

1.  Factor having a high percentage of CTA on tumor cells is presents in supernatant produced from killed or destroyed epithelial-like, fibroblast-like or lymphoblast-like cells.

2.  The factor is effective on tumor cells under the circumstances of up to the heating temperature of 56°C for 30 minutes.  It also loses CTA on tumor cells under the circumstances of more than the heating temperature of 70°C for 1 hour.

3. The molecular weight of the factor is more than of 100,000.

4. No macrophage cell line has an useful CTA.

5. The above mentioned CTA is inactive to normal cells.

From these results, it is confirmed that the characteristics of the factor are quite similar to that of TNF.

As is evident from the experiments, it is confirmed that effective TNS can be extracted from the supernatant contained killed or destroyed fibroblast, epithelial or lymphoblast-like cells. By another experiments, it is confimed that effective TNS can be extracted from the supernatant contained epithelial-like cells destroyed by a supersonic instead of a quick freezing and quick thawing.

- 16 -

## CLAIMS

1. A method of extracting a tumor necrosis factor-like substance from cells which comprises incubating at least one type of cell selected from fibroblast, epithelial and lymphoblast cells in a culture medium, killing said cells naturally in said culture medium, and extracting a tumor necrosis factor-like substance from said culture medium contained in said killed cells.

2. A method of extracting a tumor necrosis factor-like substance from cells which comprises incubating at least one type of cell selected from fibroblast, epithelial and lymphoblast cells in a culture medium, destroying said cells in said culture medium by rapid freezing and thawing, and extracting a tumor necrosis factor-like substance from said culture medium contained in said destroyed cells.

3. A method according to claim 2 in which the rapid freezing and thawing comprises supersonic treatment.